# EUROPEAN PATENT SPECIFICATION

(11) **EP 1 841 330 B1**
(45) Date of publication and mention of the grant of the patent: **16.02.2011**
(21) Application number: 05819164.4
(22) Date of filing: 14.12.2005
(51) Int. Cl.: A23L 1/29, A23L 1/305

(54) **USE OF INFANT FORMULA WITH REDUCED PROTEIN CONTENT**
VERWENDUNG VON SÄUGLINGSNAHRUNG MIT REDUZIERTEM PROTEINGEHALT
UTILISATION D'UNE PRÉPARATION POUR NOURRISSONS À CONTENU PROTÉIQUE RÉDUIT

(30) Priority: 27.12.2004 EP 04030371
(43) Date of publication of application: 10.10.2007
(73) Proprietor: Nestec S.A., 1800 Vevey (CH)
(72) Inventor: STEENHOUT, Philippe, CH-1814 La Tour-de-Peilz (CH)
(86) International application number: PCT/EP2005/056774
(87) International publication number: WO 2006/069918

(56) References cited:
- US-B1- 6 777 391
- METGES C C: "Does dietary protein in early life affect the development of adiposity in mammals?" THE JOURNAL OF NUTRITION. JUL 2001, vol. 131, no. 7, July 2001 (2001-07), pages 2062-2066, XP002369003 ISSN: 0022-3166
- FILHO J C ET AL: "Effect of protein intake on plasma and erythrocyte free amino acids and serum IGF-I and IGFBP-1 levels in rats." THE AMERICAN JOURNAL OF PHYSIOLOGY. OCT 1999, vol. 277, no. 4 Pt 1, October 1999 (1999-10), pages E693-E701, XP002369004 ISSN: 0002-9513
- KETELSLEGERS J-M ET AL: "Nutritional regulation of insulin-like growth factor-I" METABOLISM, CLINICAL AND EXPERIMENTAL, W.B. SAUNDERS CO., PHILADELPHIA, PA, US, vol. 44, October 1995 (1995-10), pages 50-57, XP004539047 ISSN: 0026-0495
- LÖNNERDAL BO: "Nutritional and physiologic significance of human milk proteins." THE AMERICAN JOURNAL OF CLINICAL NUTRITION. JUN 2003, vol. 77, no. 6, June 2003 (2003-06), pages 1537S-1543S, XP002334492 ISSN: 0002-9165

## Description

This invention relates to an infant formula with a reduced protein content.

Mother's milk is recommended for all infants. However, in some cases breast feeding is inadequate or unsuccessful or inadvisable for medical reasons or the mother chooses not to breast feed. Infant formulas have been developed for these situations. Greater knowledge of the composition of human milk affords the opportunity to design infant formulas that are closer in composition to human milk. Particular consideration has been given to devising formulas consumption of which results in growth and metabolic patterns similar to those of breastfed infants, in the hope that this will result in the development of similar health characteristics in later childhood and adulthood.

Dietary protein provides the essential amino acids necessary for protein synthesis and growth and, in infant formula, protein quality is as important as protein quantity. Infant formulas are usually based on cows' milk but the amino acid profile of cows' milk is noticeably different from that of human milk. In the past, in order to supply enough of the essential amino acids, infant formulas based on cows' milk had to have a protein content significantly higher than that of the human milk, which, in fact, has the lowest protein concentration found in any mammal. The protein content of regular whey-adapted formulas ranges from 2.1 to 2.6 g per 100 kcal, whereas the protein content of human milk ranges from 1.4 to 1.8 g per 100 kcal. Excess protein intake by infants may result in metabolic stress on immature organs.

More recently, it has been realised that if the amino acid pattern of a cow's milk-based infant formula is made closer to that of human milk, the protein content of such a formula can be reduced to resemble that of the reference.

US-B1-6777391 discloses an infant formula having 1.83g/100 kcal modifed whey, which results in plasma amino acids levels wich are closer to levels found in breast-fed infant.

It is known that the growth and metabolic parameters of breast fed and formula fed infants are not identical but the differences are currently not well understood. A key control point in nutritional regulation of growth is IGF-1, an insulin-like growth peptide synthesised by the liver which is found in human milk. It is known that formula fed infants typically display higher levels of plasma IGF-1 than breast fed infants. It is hypothesized that this may be another consequence of excessive protein intake in formula fed infants.

It has been demonstrated in infant monkeys that reducing the protein content of the formula results in a growth pattern and early age insulin and glucose metabolism more similar to that of a control breast fed group than to those of groups fed formula with higher protein contents. Further, it has been shown in human infants that the reduction in plasma IGF-1 achieved by feeding a lower protein formula in the first few months of infancy persists even after the advent of mixed feeding. As increased IGF-1 levels may result in a different body composition and an increased predisposition to obesity in later life, the regulation of IGF-1 levels in formula fed infants is a serious issue requiring further investigation.

### Summary of the Invention

It has now surprisingly been found that not only does reducing protein content in infant formula result in a reduction in plasma IGF-1 levels, it also results in the level of circulating IGF-1 over time tracking the levels observed in breast fed infants. In other words, the reduction in IGF-1 levels observed upon reduction of the protein content in infant formula is not, as was previously thought a simple step-wise reduction, but a continuous decrease over time is also observed in the first few months of life.

Accordingly, the present invention provides a method of continuously reducing the circulating level of insulin like growth factor 1 (IGF-1) in the first few months of the life of an infant by administering to an infant in need thereof a therapeutic amount of a nutritional composition comprising proteins in an amount such that the composition contains less than 2.25g of protein per 100kcal.

The invention also extends to the use of a source of proteins for the preparation of nutritional composition for administration to a human infant so as to continuously reduce the circulating level of IGF-1 in the first few months of the life of the infant wherein the composition contains less than 2.25g of protein per 100kcal.

The invention further extends to the use of a source of proteins for the preparation of nutritional composition for administration to a human infant in the first few months of the life of the infant so as to reduce the risk of development of obesity later in life wherein the composition contains less than 2.25g of protein per 100kcal.

IGF-1 is a non-specific growth factor which stimulates the growth of many tissues. Without wishing to be bound by theory, it is currently believed that a relatively high protein intake early in life stimulates secretion of IGF-1 and thereby triggers cell multiplication and accelerates maturation. The increased IGF-1 concentrations may then accelerate growth and increase adipose tissue and muscle mass thereby inducing an early adiposity rebound which is associated with an elevated risk of obesity later in childhood and even in adulthood. Further, it follows that the mitogenic effects of IGF-1 may mean that regulation of IGF-1 levels in early infancy such as may be achieved by the method of the invention may have a protective effect on the development of cancers later in life comparable to that conferred by breast milk.

### Figures

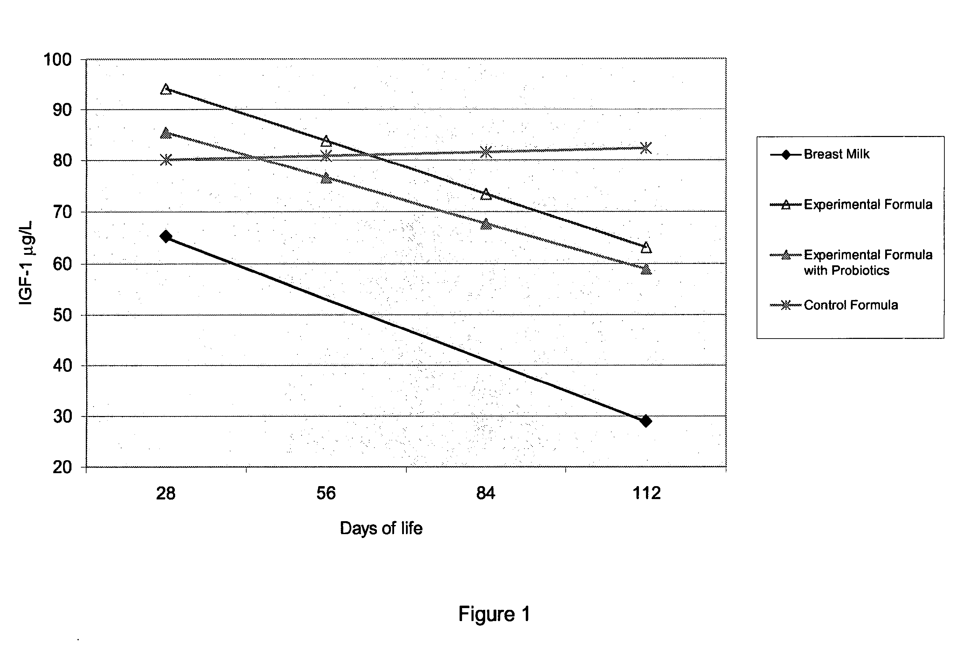

Figure 1 shows the evolution of plasma IGF-1 levels in a number of babies from day 28 to day 112 in the life of the babies.

### Detailed Description of the Invention

In this specification, the following expressions have the meanings assigned to them in the European Commission Directive 91/321/EEC of 14 May 1991 on infant formulae and follow-on formulae as follows:-
Infant: a child under the age of 12 months (Article 1.2(a));
Infant formula: a foodstuff intended for particular nutritional use by infants during the first four to six months of life and satisfying by itself the nutritional requirements of this category of persons (Article 1.2(c)).

The expression "the first few months of life" means the first four to six months of life.

The source of the protein is not believed to be critical to the present invention provided that the minimum requirements for essential amino acid content are met and satisfactory growth is ensured. Thus, protein sources based on cows' milk proteins such as whey, casein and mixtures thereof may be used as well as protein sources based on soy. As far as whey proteins are concerned, the protein source may be based on acid whey or sweet whey, whey protein isolate or mixtures thereof and may include alpha-lactalbumin and beta-lactoglobulin in whatever proportions are desired.

Preferably, however, the protein source is based on modified sweet whey. Sweet whey is a readily available by-product of cheese making and is frequently used in the manufacture of infant formulas based on cows' milk. However, sweet whey includes a component which is undesirably rich in threonine and poor in tryptophan called caseino-glyco-macropeptide (CGMP). Removal of the CGMP from sweet whey results in a protein with a threonine content closer to that of human milk. This modified sweet whey can then be supplemented with those amino acids in respect of which it has a low content (principally histidine, arginine and tryptophan). A process for removing CGMP from sweet whey is described in EP 880902 and an infant formula based on this modified sweet whey is described in WO 01/11990. Using modified sweet whey as the principal protein in the protein source enables all essential amino acids to be provided at a protein content between 1.8 and 2.0g/100kcal. Such protein sources have been shown in animal and human studies to have a protein efficiency ratio, nitrogen digestibility, biological value and net protein utilisation comparable to standard whey-adapted protein sources with a much higher protein content per 100 kcal and to result in satisfactory growth despite their reduced protein content. If modified sweet whey is used as the protein source, it is preferably supplemented by free arginine in an amount of from 0.1 to 3% by weight and/or free histidine in an amount of from 0.1 to 1.5% by weight

An example of a suitable amino acid profile for a nutritional composition to be used in the present invention is given below:-

| **Amino acid (g /16 g N)** | **Amount** |
|---|---|
| Isoleucine | 5.8 |
| Leucine | 11.9 |
| Lysine | 10.0 |
| Methionine | 2.5 |
| Cystine | 2.4 |
| Phenylalanine | 4.6 |
| Tyrosine | 4.0 |
| Threonine | 5.4 |
| Tryptophan | 2.1 |
| Valine | 5.9 |
| Arginine | 4.5 |
| Histidine | 2.5 |
| Alanine | 5.1 |
| Aspartic acid | 11.1 |
| Glutamic acid | 19.7 |
| Glycine | 2.7 |
| Proline | 7.8 |
| Serine | 5.3 |

The proteins may be intact or hydrolysed or a mixture of intact and hydrolysed proteins although intact proteins are generally preferred. However, it may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. For an extensively hydrolysed protein, the whey proteins may be subjected to triple hydrolysis using Alcalase 2.4L (EC 940459), then Neutrase 0.5L (obtainable from Novo Nordisk Ferment AG) and then pancreatin at 55°C. Alternatively, for a less hydrolysed protein, the whey may be subjected to double hydrolysis using NOVOZYMES and then pancreatin. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

Preferably the nutritional composition contains between 1.8 and 2.0g protein/100kcal, more preferably between 1.82 and 1.92g/100kcal.

Preferably the nutritional composition is an infant formula. Such a nutritionally complete composition will also contain other ingredients of the type conventionally found in infant formulas such as carbohydrates, fats, vitamins and minerals as well as semi-essential nutrients.

The preferred source of carbohydrates is lactose although other carbohydrates such as saccharose, maltodextrin, and starch may also be added. Preferably carbohydrate sources contribute between 35 and 65% of the total energy of the formula

The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula will also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B₁, vitamin B₂, vitamin B₆, vitamin B₁₂, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides, and the like. This is especially the case if the formula is provided in liquid form.

The infant formula may optionally contain other substances which may have a beneficial effect such as fibres, lactoferrin, nucleotides, nucleosides, and the like. Probiotic bacteria such as Bifidobacterium longum BB 536 and Lactobacillus rhamnosus LGG may also be included

The infant formula may be prepared in any suitable manner. For example, an infant formula may be prepared by blending together the protein source, the carbohydrate source, and the fat source in appropriate proportions. If used, emulsifiers may be included in the blend at this stage. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.

The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger.

The liquid mixture may then be cooled to about 60°C to about 85°C; for example by flash cooling. The liquid mixture may then be homogenised; for example in two stages at about 7 MPa to about 40 MPa in the first stage and about 2 MPa to about 14 MPa in the second stage. The homogenised mixture may then be further cooled and any heat sensitive components; such as vitamins and minerals may be added. The pH and solids content of the homogenised mixture is conveniently standardised at this point.

If it is desired to produce a powdered infant formula, the homogenised mixture is transferred to a suitable drying apparatus such as a spray drier or freeze drier and converted to powder. The powder should have a moisture content of less than about 5% by weight.

If it is desired to produce a liquid infant formula, the homogenised mixture is filled into suitable containers; preferably aseptically. However, the liquid infant formula may also be retorted in the container. Suitable apparatus for carrying out filling of this nature is commercially available. The liquid infant formula may be in the form of a ready to feed formula having a solids content of about 10 to about 14% by weight or may be in the form of a concentrate; usually of solids content of about 20 to about 26% by weight.

An example of the composition of a suitable infant formula to be used in the present invention is given below:-

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B 12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |

The following example is given by way of illustration only and should not be construed as limiting the subject-matter of the present application.

### Example

### Influence of protein content on plasma IGF-1 levels in the first four months of life

This example demonstrates the effect of the protein content of an infant formula used as the sole source of nutrition for a group of infants for the first four months of their life on their plasma IGF-1 levels.

A prospective, randomized, controlled, blinded study of three groups in parallel was carried out at the University of Iowa (Lora N. Thomas Metabolism Ward) and at its annex in Cedar Rapids, Iowa in accordance with the principles established in the 1964 Declaration of Helsinki (as amended) and with the approval of the University of Iowa Committee on Research Involving Human Subjects. Infants whose mothers had decided not to breast feed were recruited at the maternity units of two hospitals in Iowa City and two in Cedar Rapids and randomly assigned to one of three groups. The control group was fed a partially hydrolysed infant formula with a protein content of 2.39g/100kcal available commercially in the United States under the trade mark Good Start ®. Of the other two groups one was fed an experimental partially hydrolysed formula with a protein content of 1.92g/100kca1 and the other was fed a similar experimental formula with a protein content of 1.89g/100kcal but with the addition of a probiotic bacterium, Bifidobacterium lactis Bb12 (obtainable from Christian Hansen, Denmark) in an amount of 3.6 x 10⁷ colony forming units per gram of formula powder (equivalent to 4.8 x 10⁹ CFU/litre). In all formulas, the protein consisted of partially hydrolysed whey proteins. The experimental formulas were derived from sweet whey and the control formula from acid whey. The formulas were supplied packed in metal cans with their identity marked by a colour coding known only to the investigating staff.

The compositions of the formulas are summarised in the following table:-

| Concentrations are per litre unless otherwise stated | Experimental | Experimental with probiotic | Control |
|---|---|---|---|
| Energy (kcal) | 680 | 680 | 670 |
| Protein (g) | 13.1 | 12.8 | 16.0 |
| -g/100kcal | 1.92 | 1.89 | 2.39 |
| Fat (g) | 34.6 | 34.6 | 34.6 |
| Lactose (g) | 55.3 | 55.3 | 55.3 |
| Maltodextrin (g) | 23.7 | 23.7 | 21.5 |
| Na (mmol) | 8.7 | 8.7 | 7.0 |
| K (mmol) | 16.7 | 16.7 | 16.5 |
| Cl (mmol) | 13.0 | 13.0 | 11.0 |
| Ca (mg) | 423 | 446 | 506 |
| P (mg) | 244 | 240 | 279 |
| Mg (mg) | 48.1 | 48.3 | 50.2 |
| Mn (µg) | 40 | 40 | 50 |
| Se (µg) | 13 | 13 | 0 |
| Fe (mg) | 7.1 | 7.3 | 12.9 |
| I (µg) | 100 | 100 | 54 |
| Cu (mg) | 0.645 | 0.643 | 0.733 |
| Zn (mg) | 7.3 | 7.35 | 6.75 |

The amino acid profiles of the formulas were as follows:-

| Amino acids (g/l) | Experimental | Control |
|---|---|---|
| Alanine | 0.72 | 0.88 |
| Arginine | 0.65 | 0.43 |
| Aspartic Acid | 1.61 | 1.97 |
| Cysteine | 0.42 | 0.41 |
| Glutamic Acid | 2.42 | 3.16 |
| Glycine | 0.25 | 0.35 |
| Histidine | 0.40 | 0.32 |
| Isoleucine | 0.80 | 1.10 |
| Leucine | 1.73 | 1,90 |
| Lysine | 1.40 | 1.54 |
| Methionine | 0.32 | 0.35 |
| Phenylalanine | 0.47 | 0.56 |
| Proline | 0.66 | 1.06 |
| Serine | 0.57 | 0.89 |
| Threonine | 0.76 | 1.30 |
| Tryptophan | 0.31 | 0.29 |
| Tyrosine | 0.42 | 0.40 |
| Valine | 0.77 | 1.05 |
| Peptide Size distribution (%) | | |
| >5000 Da | 1.8 | 8.7 |
| 2500 - 5000 Da | 6.9 | 8.9 |
| 1000 - 2500 Da | 28.6 | 27.8 |
| <1000 Da | 62.8 | 64.5 |

140 infants were recruited with the intention that at least 28 in each group would complete the study. The subjects were normal, healthy, full-term infants 6 to 9 days old. Approximately equal numbers of males and females were recruited. The study was complete for each infant after 112 days. The infants were fed their assigned formula ad libitum as the sole source of nutrition. Data was collected from birth records, on enrolment, and at visits on Day 14 (± 2), 28 (± 2), 42 (± 2), 56 (± 4), 84 (± 4), and 112 (± 4).

The following measurements were made and records kept:-

Growth was assessed by anthropometric measurements including weight, recumbent length, and head circumference. Food intake was recorded and tolerance of the formulas was assessed using two day tolerance records, recording stools and feeding related behaviours. Capillary blood samples were drawn at Days 28, 56, 84, and 112 and laboratory analyses of plasma samples were conducted for albumin, total protein, plasma urea nitrogen, haemoglobin, plasma free amino acids (days 28 and 112 only), IGF-1 and leptin.

No significant difference in average weight gain, average length gain and average head circumference gain per day was found between the three formulas. In protein status, there is no significant difference except for plasma urea nitrogen which is higher for the control formula (p<0.001). No significant difference was found in plasma leptin levels. However, reference to Figure 1 of the drawings shows that a significant difference was observed over the period of the study in the evolution of plasma IGF-1 levels for infants fed the control formula compared to infants fed the experimental formulas.

In infants fed the experimental formulas, IGF-1 levels decreased significantly (p=0.013) between day 28 and day 112. IGF-1 levels averaged 79.3 ± 34.0 (mean±SD, N=35) at 28 days and 58.9 ± 37.8 (N=41) at 112 days of age. On the other hand, in infants fed the control formula IGF-1 levels did not decrease. In these infants, IGF-1 levels averaged 77.5 ± 31.0 (N=21) at 28 days and 80.8 ± 37.8 (N=27) at 112 days of age. The addition of a probiotic had no effect on IGF-1 levels. These results are based on blood samples taken from 88 infants being the number of infants that completed the study and that did not receive other infant formulas or weaning foods during the study period.

These results are presented graphically in Figure 1 which shows the plasma IGF-1 levels in µg/l as determined from blood samples taken at the day 28, 56, 84 and 112 visits. It may be seen that not only were the IGF-1 levels in infants fed the experimental formulas lower than in those fed the control formula for most of the study period but also the IGF-1 levels in infants fed the experimental formulas decreased steadily over the study period and, moreover, at a rate which was substantially comparable to the decrease in plasma IGF-1 levels in breast fed infants over the same period (data from a previous study). By comparison, the plasma IGF-1 level in infants fed the control formula hardly changed over the period of the study. In view of the role played by IGF-1 in the regulation of growth, this could further provide a mechanism to reduce the risk of obesity later in life in formula fed infants.

## Claims

1. The use of a source of proteins for the preparation of nutritional composition for administration to a human infant so as to continuously reduce the circulating level of IGF-1 in the first few months of the life of the infant wherein the composition contains less than 2.25g of protein per 100kcal.

2. The use of a source of proteins for the preparation of nutritional composition for administration to a human infant in the first few months of the life of the infant so as to reduce the risk of development of obesity later in life wherein the composition contains less than 2.25g of protein per 100kcal.

3. The use of Claim 1 or 2, wherein the composition comprises between 1.8 and 2.0 g of protein per 100kcal.

4. The use of any preceding claim, wherein the source of proteins is cows' milk

5. The use of any preceding claim, wherein the source of proteins is sweet whey protein from which caseino-glyco-macropeptide has been removed.

6. The use of Claim 5 wherein the composition additionally includes free arginine in an amount of from 0.1 to 3.0 % by weight and/or free histidine in an amount of from 0.1 to 1.5 % by weight.

7. The use of any preceding claim, wherein the proteins are intact.

8. The use of any of Claims 1 to 7, wherein the proteins are partially hydrolysed.

9. The use of Claim 8 wherein the degree of hydrolysis of the proteins is between 2 and 20%.

10. The use of any preceding claim, wherein the nutritional composition is an infant formula.

## Patentansprüche

1. Verwendung einer Proteinquelle zur Herstellung einer Nahrungszusammensetzung zur Verabreichung an menschliche Kinder um so das sich in Umlauf befindliche Niveau an IGF-1 in den ersten Lebensmonaten des Kindes kontinuierlich zu verringern, wobei die Zusammensetzung weniger als 2,25g Protein pro 100 kcal enthält.

2. Verwendung einer Proteinquelle zur Herstellung einer Nahrungszusammensetzung zur Verabreichung an ein menschliches Kind in den ersten Lebensmonaten des Kindes, um so das Risiko der Entwicklung von Übergewicht später im Leben zu verringern, wobei die Zusammensetzung weniger als 2,25g Protein pro 100 kcal enthält.

3. Verwendung nach Anspruch 1 oder 2, wobei die Zusammensetzung zwischen 1,8 und 2,0g Protein pro 100 kcal umfasst.

4. Verwendung nach einem vorhergehenden Anspruch, wobei die Proteinquelle Kuhmilch ist.

5. Verwendung nach einem vorhergehenden Anspruch, wobei die Proteinquelle Süßmolkenprotein ist, aus dem Casein-Glyco-Macropeptid entfernt worden ist.

6. Verwendung nach Anspruch 5, wobei die Zusammensetzung freies Arginin in einer Menge von 0,1 bis 3,0 Gewichtsprozent und/oder freies Histidin in einer Menge von 0,1 bis 1,5 Gewichtsprozent einschließt.

7. Verwendung nach einem vorhergehenden Anspruch, wobei die Proteine intakt sind.

8. Verwendung nach einem der Ansprüche 1 bis 7, wobei die Proteine partiell hydrolisiert sind.

9. Verwendung nach Anspruch 8, wobei der Hydrolysegrad der Proteine zwischen 2 und 20% beträgt.

10. Verwendung nach einem vorhergehenden Anspruch, wobei die Nahrungszusammensetzung eine Säuglingsanfangsnahrung ist.

## Revendications

1. Utilisation dune source de protéines pour la préparation dune composition nutritionnelle pour l'administration à un enfant humain afin de réduire de manière continue le niveau de IGF-1 en circulation dans les quelques premiers mois de la vie de l'enfant, la composition contenant moins de 2,25 g de protéines pour 100 kcal.

2. Utilisation dune source de protéines pour la préparation dune composition nutritionnelle pour l'administration à un enfant humain dans les quelques premiers mois de la vie de l'enfant afin de réduire le risque de développement de l'obésité plus tard dans la vie, la composition contenant moins de 2,25 g de protéines pour 100 kcal.

3. Utilisation selon la revendication 1 ou 2, la composition comprenant entre 1,8 et 2,0 g de protéines pour 100 kcal.

4. Utilisation selon l'une des revendications précédentes, la source de protéines étant du lait de vache.

5. Utilisation selon l'une des revendications précédentes, la source de protéines étant de la protéine lactosérique sucrée, de laquelle le caséino-glyco-macropeptide a été enlevé.

6. Utilisation selon la revendication 5, la composition incluant en outre de l'arginine libre dans une quantité allant de 0,1 à 3,0 % en poids et/ou de l'histidine libre dans une quantité allant de 0,1 à 1,5 % en poids.

7. Utilisation selon l'une des revendications précédentes, les protéines étant intactes.

8. Utilisation selon l'une des revendications 1 bis 7, les protéines étant partiellement hydrolysées.

9. Utilisation selon la revendication 8, le degré d'hydrolyse des protéines étant compris entre 2 et 20 %.

10. Utilisation selon l'une des revendications précédentes, la composition nutritionnelle étant une formule pour enfant en bas âge.
